# EUROPEAN PATENT SPECIFICATION

(11) **EP 2 304 442 B1**
(45) Date of publication and mention of the grant of the patent: **14.11.2012**
(21) Application number: 09761735.1
(22) Date of filing: 10.06.2009
(51) Int. Cl.: G01N 33/66

(54) **BLOOD SERUM MARKER FOR DETECTION AND DIAGNOSIS OF ALZHEIMER'S DISEASE**
BLUTSERUMMARKER ZUM NACHWEIS UND ZUR DIAGNOSE VON MORBUS ALZHEIMER
MARQUEUR DE SÉRUM SANGUIN POUR LA DÉTECTION ET LE DIAGNOSTIC DE LA MALADIE D'ALZHEIMER

(30) Priority: 11.06.2008 EP 08158010
(43) Date of publication of application: 06.04.2011
(73) Proprietor: VIB VZW, 9052 Gent (BE); Universiteit Gent, 9000 Gent (BE)
(72) Inventor: CHEN, Cuiying, B-9860 Balegem (BE); LIBERT, Claude, B-9700 Oudenaarde (BE)
(86) International application number: PCT/EP2009/057162
(87) International publication number: WO 2009/150172

(56) References cited:
- WO-A2-2007/044471
- WOUTER LAROY; ROLAND CONTRERAS; NICO CALLEWAERT: "Glycome mapping on DNA sequencing equipment" NATURE PROTOCOLS, NATURE PUBLISHING GROUP, UK, vol. 397, no. 1, 27 June 2006 (2006-06-27) , pages 397-405, XP001538561 ISSN: 1750-2799
- AKASAKA-MANYA KEIKO; MANYA HIROSHI; SAKURAI YOKO; WOJCZYK BOGUSLAW S; SPITALNIK STEVEN L; ENDO TAMAO: "Increased bisecting and core-fucosylated N-glycans on mutant human amyloid precursor proteins." GLYCOCONJUGATE JOURNAL NOV 2008, vol. 25, no. 8, 3 June 2008 (2008-06-03), pages 775-786, XP002542852 ISSN: 0282-0080

## Description

The present invention relates to a blood marker for early detection and diagnosis of Alzheimer's disease. More specifically, it relates to an analysis of the protein glycan profile in the blood, and the identification of specific peak ratio's when compared to healthy aged matched subjects that are typical for Alzheimer's dementia.

Alzheimer's disease (AD), the most common type of dementia, is a progressive, degenerative brain disease, accounting for 65-70% of all cases (Blennow et al., 2006). The incidence of AD increases substantially after the age of 70 and represents a major health concern and economic burden worldwide. Though AD primarily affects older people, it is not a normal part of aging and is not something that inevitably happens in later life (Wilson, 2008). Neither preventive nor curative measures for AD are currently available because the cause of the disease is unknown.

The main pathological characteristics of AD are defined mainly by extracellular β-amyloid protein (Aβ) deposition in the brain parenchyma and cerebral blood vessels and by the presence of neurofibrillary tangles. These deposits are derived from dysfunctional cleavage of the ubiquitous amyloid precursor protein (APP) Selkoe and Schenk, 2003). Abnormal levels of an β-amyloid peptide (such as Aβ-42) and the tau protein in cerebrospinal fluid (CSF) have been found in patients with AD, and thus these two proteins have been investigated for their diagnostic utility (Hampel et al., 2003; Maddalena et al., 2003; Ibach et al., 2006). Aβ-42 stands for a subtype of amyloid beta peptide that is produced following the metabolism of an amyloid precursor protein. Low levels of Aβ-42 in the CSF have been associated with AD, perhaps because Aβ-42 is deposited in the amyloid plaques instead of remaining in solution. The tau protein is a microtubule-associated molecule that is found in the neurofibrillary tangles that are typical of AD. Tau protein is related to degenerating and dying neurons, and high levels of total tau (t-tau) proteins in the CSF have been associated with AD. Recently, phosphorylated tau protein (p-tau) was recently investigated as a more specific marker for AD (Hampel et al., 2003; Buerger et al., 2002). The combination of elevated CSF t-tau or p-tau protein and diminished CSF Aβ-42 are the only biomarkers with enough sensitivity and specificity to serve as useful diagnostic biomarker capable of distinguishing AD from other dementias in the early stages (Growdon, 1998). However, there is significant overlap in the values of CSF biomarkers in AD and non-AD patients, which limits their usefulness. Neural thread protein is another protein that is associated with the neurofibrillary tangles of AD, and both CSF and urine levels of this protein have been investigated as a biochemical markers of AD. Although the apolipoprotein E ε4 allele (ApoE ε4) is a genetic risk factor (Myers et al, 1996), we recently found that is no associations between the levels of CSF biomarkers and ApoE ε4 in AD (Engelborghs et al., 2007a).

Several investigators have tried to use structure biomarker (CT or MRI measures) to help in the a diagnosis of AD (Heckemann et al., 2008). Diagnostic decisions in clinical imaging of suspected dementia patients currently rely almost exclusively on visual image interpretation, which can lead to uncertainty when some of the changes resemble those of normal aging. Various indices for AD have been developed with different sensitivities and specificities (De Carli et al., 1990; Nagy et al., 1999; Mattman et al., 1997; De Leon et al., 1997; Frisoni et al., 2002; Brand et al., 2007; Barkhof et al., 2007; Blasko et al., 2008).

No specific "blood test" or imaging technique is currently used for the definitive diagnosis of AD (Blasko et al., 2006). The only way to ascertain that someone had AD is by microscopic examination of a sample of brain tissue after death (Dubois et al., 2007). Before death, only a "possible" or "probable" diagnosis of the disease can be made using current criteria (Knopman et al., 2001; Waldemar et al., 2007). The reported accuracy of clinical diagnosis of AD is quite variable, and a reliable biochemical marker is needed to help in the accurate and early diagnosis of AD (Galton, 2000). Current therapies are initiated only after diagnosis; their modest benefit, in part, may be explained by the fact that some irreversible brain damage had already occurred by the time dementia is recognized (van Marum, 2008). The development of valid and reliable biomarkers for AD will not only aid clinicians in recognizing the disease in its earliest symptomatic stages, but may also may help identify the illness before dementia or other symptoms appear. The detection of preclinical AD will be especially important should effective disease-modifying therapies be developed to allow optimal intervention.

Protein glycosylation, the most common co-translational modification, plays important biological roles by influencing the functions of glycoproteins (Kukuruzinska and Lennon, 1998). Glycoproteins are important for initiation of various cellular recognition signals that are essential for the maintenance of the ordered social life of each cell within a multi-cellular organism (Raman et al., 2005). Because the biosynthesis of glycans is not controlled by interaction with a template but depends on the complicated concerted action of glycosyltransferases, the structures of glycans are much more variable than those of proteins and nucleic acids, and they can be easily altered by the physiological conditions of the cells.

WO 2007044471 discloses methods and products related to the improved analysis of carbohydrates, and indicates that the profiles can be used in diagnosis of diseases, such as neurodegenerative diseases including Alzheimer's. However, the application does not disclose specific changes and has no indication of which glycan structure(s) could be used for the diagnosis of Alzheimer disease. Laroy et al (2006) disclose a method for glycome mapping on DNA sequencing equipment, but there is no indication that the glycome analysis can be used for the detection of follow up of Alzheimer. Akasaka-Manya et al. (2008) disclosed that C17 cells, transfected with either the wild type or mutant amyloid precursor protein gene, and indicated that there is an increase of bisecting and core-fucosylated N-glycans on the mutant form. However, there is no indication that the same effect is seen in vivo, neither that the analysis can be carried out on serum of patients.

Our recent study showed that the level of N-glycans in serum changes during healthy aging (Vanhooren et al., 2007). Surprisingly we found that there is also a difference in glycan profile between Alzheimer's disease patients and the age matched healthy control. Even more surprisingly, the profile allows to discriminate between Alzheimer's dementia and non-Alzheimer's dementia.

A first aspect of the invention is a method for the detection of Alzheimer's disease in female human subjects, comprising the determination of bigalactosylated, care-α+1,6-fucosylated biantennary glycan structures (NA2F) in serum and comparing said concentration with the concentration of a healthy reference subject. Healthy reference as used here, means that the subject does not suffer of dementia. For the determination of the amount of NA2F and/or NA3Fb, any method know to the person skilled in the art can be used. As a non limiting example, the amount can be determined by ELISA, using specific antibodies, of by the use of specific lectins. Preferably the concentration is determined by determining the specific peak height of the peaks in the profile generated by DSA-FACE.

Another aspect of the invention is the use of the analysis of the glycan structure, according to the invention, to follow up the development of Alzheimer's disease and/or evolution during treatment of Alzheimer's disease. The glycan analysis according to the invention is not only useful to follow the effect of the treatment in time, but it can also be used to evaluate the efficacity of a candidate compound for the treatment of Alzheimer disease.

### BRIEF DESCRIPTION OF THE FIGURES

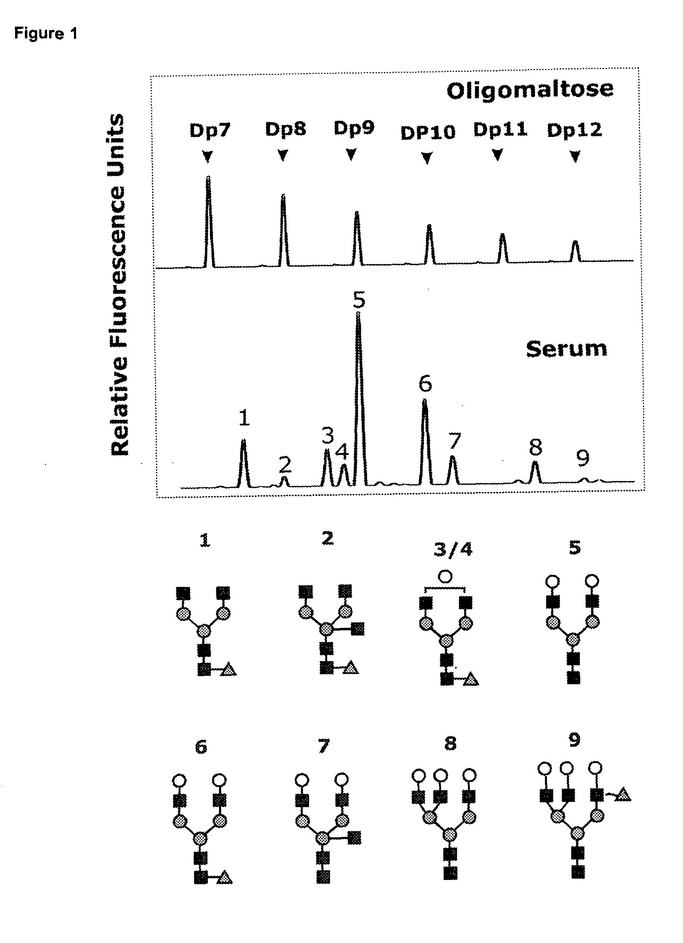

Figure 1: The malto-oligosaccharides are shown at the top as sugar mass reference. The number of glucose units (DP, degree of polymerization) in these structures is indicated. A typical desialylated N-glycan profile is shown for total serum proteins. The structures of the N-glycan peaks are shown below the panels.
   Peak 1 is an agalactosylated, core-α-1,6-fucosylated biantennary glycan (NGA2F), peak 2 is an agalactosylated, core-α-1,6-fucosylated bisecting biantennary (NGA2FB), peak 3 and 4 are mono galactosylated, core-α-1,6-fucosylated biantennary (NG1A2F), peak 5 is a bigalactosylated, biantennary glycan (NA2), peak 6 is a bigalactosylated, core-α-1,6-fucosylated biantennary (NA2F), peak 7 is a bigalactosylated, core-α-1,6-fucosylated bisecting biantennary (NA2FB), peak 8 is a trigalactosylated, tri-antennary (NA3) and peak 9 is a branching α-1,3-fucosylated trigalactosylated tri-antennary (NA3F(b)). The symbols used in the structural formulas are: ■ N-acetyl-glucosamine (GlcNAc); ↓ β-linked galactose; ↖ α-1,3/6-linked fucose; ● α/β-linked mannose.
Figure 2: N-glycan values of total serum N-glycans from two controls (age <60 and 60 plus) and two dementia groups (AD and non-AD) are shown as peaks. The vertical axis represents the 95% confidence interval for mean of the percentage of total N-glycan peak height:
Figure 3: N-glycan value of total serum peak 9 (NA3Fb) from the females of controls (n=99 of =<60 years; n=50 of >60 years) and dementias (n=79). The vertical axis represents the 95% confidence interval for mean of the percentage of total N-glycan peak height. The differences between the dementia group and the 60+ year old group is statistically significant (p = 0.006).
Figure 4: Receiver operating characteristic (ROC) curve for prediction of clinically significant for detection of dementia female group using the values of peak 9 (NA3Fb). Area under the curve (AUC) shows the diagnosis power of peak 9 (0.866 ± 0.035).
Figure 5: N-glycan value of total serum peak 6 (NA2F) from the females of controls (n=50 of =<60 years; n=25 of >60 years), AD (n=24) and non-AD (n=13) groups. The level of N-glycan concentration from the AD is comparable to the age group of age <60 years, 60 plus years and non-AD patients. The vertical axis represents the 95% confidence interval for mean of the percentage of total N-glycan peak height. The statistical significance of differences between AD and 60+ years control, between AD and non-AD group is indicated as p value.
Figure 6: Receiver operating characteristic (ROC) curve for prediction of clinically significant for detection of AD female group using the values of peak 6 (NA2F). Area under the curve (AUC) shows the diagnosis power of peak 6 (0.902 ± 0.031).
Figure 7: Typical glycan profile for a wild type mouse (W) and for a R406W mutant Tau transgenic mouse (TAU). Peaks are labeled; P1, P5 and P6 are identical to peaks occurring in human, pm peaks are mouse specific.
Figure 8: Evolution of the peaks P1, pma and P5 in function of age, for wild type mice (W) and R406W mutant Tau transgenic mice (PTT). The age is indicated in months.

### EXAMPLES

### Material and methods to the examples

### Study population

Serum samples from 79 demented patients whose diagnosis was confirmed by autopsy were used in this study. All sera samples were selected from the Biobank, Institute Born-Bunge, Antwerp, Belgium. Two control groups were included: 50 samples from people older than 60 years (from the Biobank, Institute Born-Bunge) and 100 samples from people 20 to 60 years old (from the Red Cross, Ghent). Age and gender were recorded. For subjects with dementia, we also recorded the scores of the Mini-Mental State Examination (MMSE) (Folstein et al., 1975) at the time of CSF sampling, year of clinical diagnosis, clinical and pathological diagnoses, year of death, date of autopsy and type of sampling tube. The study was approved by the local ethics committee (CME Middelheim).

### Mice samples

Serum samples from R406W mutant Tau transgenic mice and wild type mice with the same genetic background were obtained from Remynd, Belgium. Samples were obtained from mice at different age, to exclude possible age effects.

### Clinical diagnostic criteria

All dementia patients were diagnosed according to strict clinical diagnostic criteria (Engelborghs et al. 2007b). The diagnosis of probable AD was established using NINCDS-ADRDA criteria (McKhann et al., 1984). All patients also fulfilled the Diagnostic and Statistical Manual of Mental Diseases (DSM-IV) criteria (APA, 1994). Mixed dementia (MXD) was diagnosed when patients fulfilled the criteria of probable AD according to NINCDS/ADRDA criteria and, in addition, displayed cerebrovascular disease (CVD) on brain CT and/or MRI but did not meet the criteria for relevant CVD according to NINDS-AIREN criteria for vascular dementia (Roman et al., 1993). In that way we excluded multiple large-vessel infarcts, strategically placed infarcts, multiple basal ganglia and white matter lacunes or extensive white matter lesions. Vascular dementia (VAD) was diagnosed according to the NINDS-AIREN criteria of vascular dementia (Roman et al., 1993). For the diagnosis of probable frontotemporal dementia (FTD) and progressive non-fluent aphasia (PPA), the criteria described by Neary et al. (1998) were applied. An extensive neuropsychological assessment revealing a characteristic neurocognitive profile of disproportionate executive dysfunction indicating frontal lobe involvement and brain perfusion SPECT were used to support the clinical diagnosis of FTD as described earlier Pickut et al., 1997). Dementia with Lewy bodies (DLB) was diagnosed by applying the clinical diagnostic criteria of McKeith, et al. (1996). Parkinson's disease dementia (PDD) was diagnosed when patients with idiopathic Parkinson's disease (PD) developed dementia following a dementia-free interval of at least two years. The criteria for the diagnosis of idiopathic PD included the presence of at least two out of four motor manifestations that characterize the disease (resting tremor, bradykinesia, muscular rigidity and impaired postural reflexes) and an insidious onset (Hoehn and Yahr, 1967). Creutzfeldt-Jakob disease (CJD) was diagnosed according to the criteria of Weber (2000). In one case, clinical differential diagnosis was Gerstmann-Straussler-Scheinker disease or spinocerebellar ataxia as described in detail by Fujigasaki et al. (2001). The inclusion criteria for the control group were: (1) no neurological or psychiatric antecedents and (2) no organic disease involving the central nervous system following extensive clinical examination. The age-matched control group (age >60, *n* = 50) consisted of patients with mechanical low back pain requiring a selective lumbar radiculography, patients with disorders of the peripheral nervous system (polyneuropathy, peripheral facial nerve palsy) and patients with subjective complaints in whom disorders of the central and peripheral nervous system were ruled out by an extensive clinical work-up.

### Pathological criteria

All diagnoses were established by the same neuropathologist who was blinded for the cerebrospinal fluid (CSF) results. For AD patients, the neuropathological criteria of Braak and Braak (1991) and of Jellinger (1998) were applied, whereas FTD patients were neuropathologically diagnosed according to Jackson and Lowe (1988) and Markesbery (1998). The pathological criteria of Kosaka et al. (1988) were applied for diagnosing DLB. MXD and VAD were diagnosed according to Markesbery (1998).

### Cerebrospinal fluid analysis

CSF analysis was performed at the R&D facilities of Innogenetics NV (Ghent, Belgium) following relabeling of the CSF vials (01-001 to 01-100; control samples: 01-101 to 01- 200). Before analysis, samples were randomized to obtain a proportional distribution of samples from controls and demented patients on each microtiter plate (40 samples per plate). The laboratory technician was blinded for the expected test outcome in terms of clinical and definitive pathological diagnoses when performing and interpreting the tests.

CSF levels of Aβ_₁₋₄₂, T-tau and P-tau181P were determined with commercially available single-parameter ELISA kits [respectively, INNOTEST® β-AMYLOID(1-42), INNOTEST® hTAU Ag, INNOTEST® PHOSPHOTAU(181P)]; Innogenetics, Ghent, Belgium). With each assay, the clinical samples, together with a blank (sample diluent), the (prepared) calibrator solutions and the appropriate controls, were tested with strict adherence to the test instructions provided in the kit inserts. All samples were run in duplicate.

If the intra-assay coefficient of variation was greater than 30% (calculated as (max - min)×100/average), or if the concentrations obtained were out-of-range (OD values not between mean OD values of highest and lowest calibrator concentration), samples were retested (by extension of the calibrator concentration range for some samples). Dilution of samples that gave values above the highest calibration concentration for possible reanalysis was not performed, as it is not recommended in the manufacturer's instructions. The concentration ranges of the test kits are described in the package inserts (P-tau181P: 15.6-500 pg/ml, T-tau: 75-1200 pg/ml, Aβ_₁₋₄₂: 125-2000 pg/ml).

### Processing blood samples for protein N-glycome analysis

The N-glycans attached to the proteins in 2 µl of serum were released, labeled, and analyzed as described (Vanhooren et al., 2008; Liu et al., 2007). Labeled N-glycans were analyzed by DNA Sequencer Assisted Fluorophore Assisted Carbohydrate Electrophoresis (DSA-FACE) technology, using a capillary electrophoresis (CE)-based ABI3130 sequencer. Data were analyzed with the GeneMapper v3.7 software (Applied Biosystems, Foster City, CA). We measured the heights of the peaks that were detected in all the samples, obtained a numerical description of the profiles, and analyzed these data with SPSS 12.0 statistical software.

### Statistical analysis

Statistical analyses were performed with SPSS for Windows software (SPSS, Chicago, IL, USA). Results are presented as means ±SD. All reported p-values are two-tailed, using a t-test for independent samples. Pearson's coefficients of correlation (with 95% confidence intervals and their associated probability, p) were used to evaluate the relationships between parameters. The Receiver Operating Characteristics (ROC) curve was used as an index of accuracy; values close to 1.0 indicate high diagnostic accuracy.

### Example 1: Description of the study population

The demographic, main clinical and neuropathological data of the patients are summarized in Table 1. Clinical diagnosis at CSF sampling in the dementia patients consisted of 48 AD patients (n=24 females, n=24 males). The other dementias including FTD, DLB and VAD, were pooled as a group of non-AD (n=13 female, n=18 male).

**Table 1. Clinical and neuropathological data**

| group | | age | MMSE score | T-tau | P_tau | Aß-42 |
|---|---|---|---|---|---|---|
| age =<60 yrs | Mean | 40.202 | | | | |
| | N | 99 | | | | |
| | Std. Deviation | 14.1407 | | | | |
| age >60 yrs | Mean | 77.999 | 27.8 | 447 | 63.5 | 599 |
| | N | 50 | 10 | 2 | 2 | 2 |
| | Std. Deviation | 5.0656 | 1.398 | 48.08326 | 7.77817 | 69.29646 |
| AD | Mean | 79.979 | 10.48 | 823.6957 | 83.4217 | 361.413 |
| | N | 48 | 40 | 46 | 46 | 46 |
| | Std. Deviation | 8.6134 | 5.435 | 751.9652 | 41.30617 | 103.4179 |
| non-AD | Mean | 74.71 | 12.88 | 554.2414 | 58.1828 | 504.2759 |
| | N | 31 | 25 | 29 | 29 | 29 |
| | Std. Deviation | 9.6305 | 9.198 | 538.0035 | 34.73765 | 171.7263 |

### Example 2: Altered N-glycan profiles in the female dementias

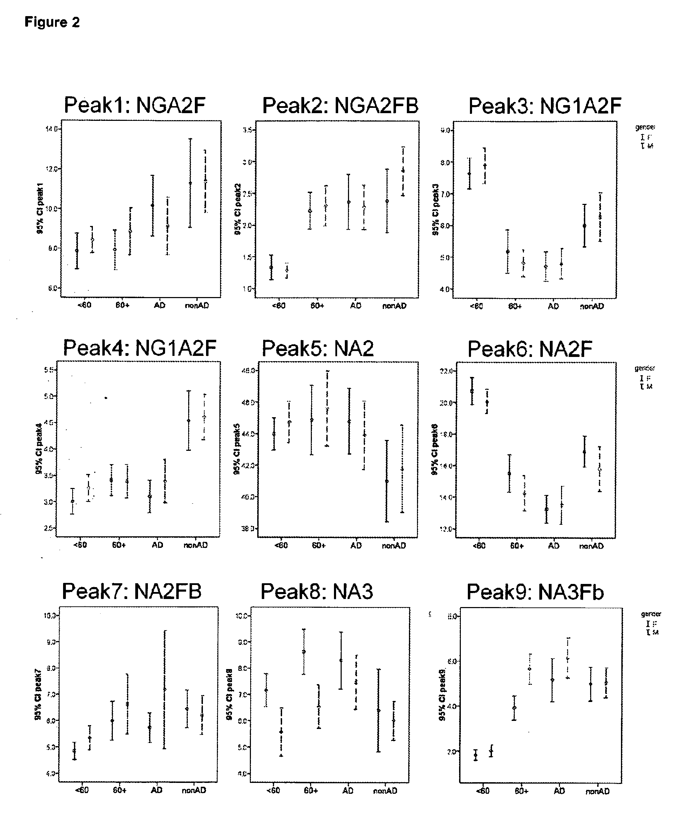

We used DSA-FACE to determine the N-glycome profiles of desialylated sera obtained from dementia patients with AD (n=48) and non-AD (n=31). We also analyzed serum from healthy donors (n=150). The glycan fingerprint is shown in Figure 1. The individual N-glycan structures are shown in Figure 1 as well. The N-glycans detected in serum are represented as peaks and the relative concentration was quantified by normalizing its height to the sum of the heights of all peaks in the profile (Figure 2).

The sizes of the peaks, which represent the relative concentrations of the oligosaccharide structures, were analyzed statistically for correlation between glycans and dementias. Two glycan structures, NA2F (peak 6) and NA3Fb (peak 9), were found to related dementia, but more pronounced in females.

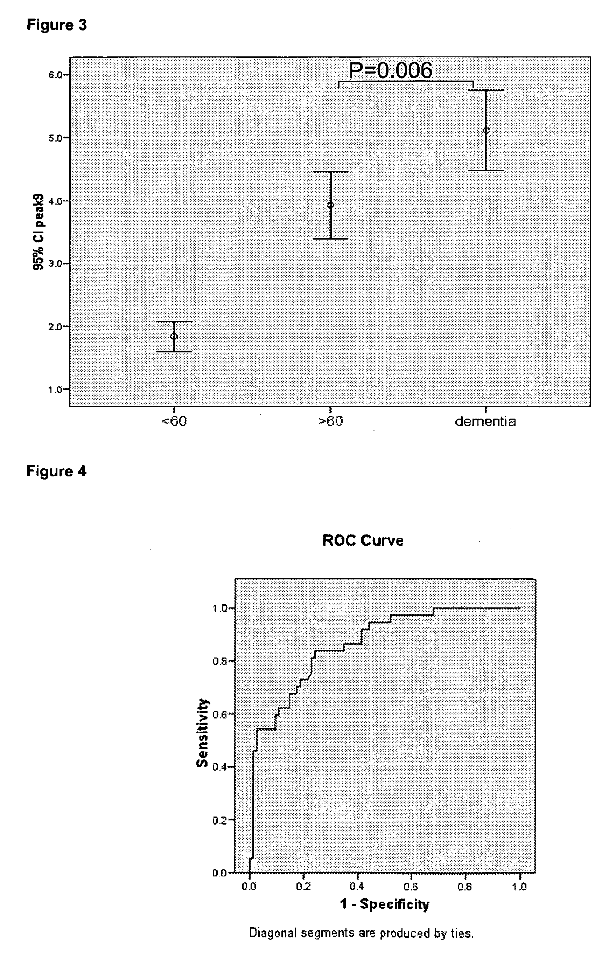

The level of peak 9 (NA3Fb) was significantly higher (p<0.006) in the female dementia patients (AD and non-AD) compared to age-matched controls (Fig. 3). ROC curve analysis showed an accuracy of 86.6% ± 3.5% for peak 9 with a 90% of specificity and 60% of sensitivity for identifying dementia patients from controls (Fig. 4).

### Example 3: Decreasing the concentration of NA2F in female AD patients

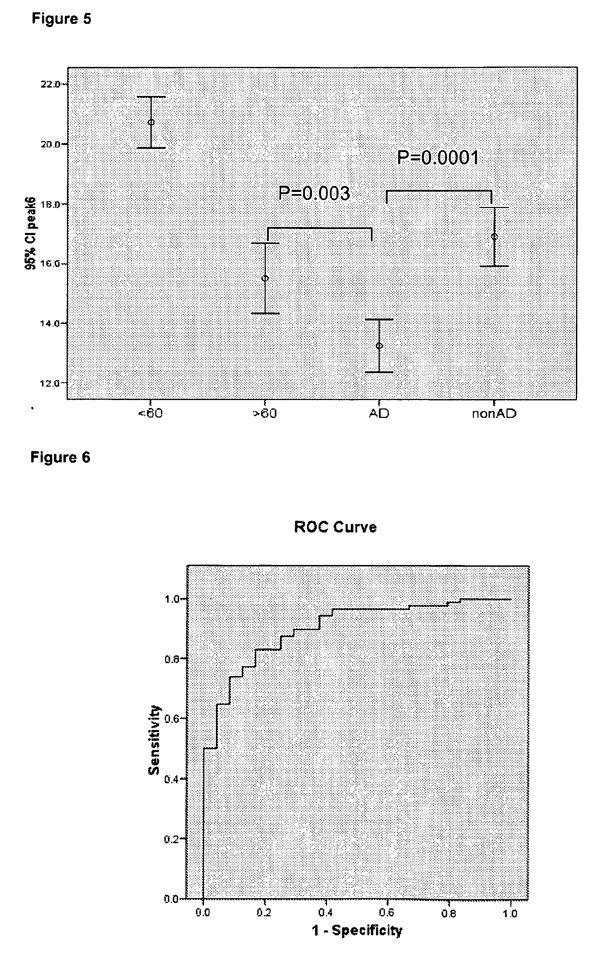

The level of peak 6 (NA2F) was substantially decreased only in AD patients, not in the non-AD and control groups (Fig. 5). ROC curve analysis showed an accuracy of 90.2% ± 3.1% for peak 6 with a 90% of specificity and 70% sensitivity for identifying AD from and non-AD and controls (Fig. 6).

### Example 4: The correlation of glycans with MMSE stage and CSF markers

Table 2 showed correlation glycan markers with CSF marker and MMSE score in the female dementias and controls. In this study, we did not found any correlation between CSF markers and MMSE score, and between β-amyloid peptide and p-tan or t-tau protein in the dementias or AD patients (including female and male). Only correlation was found is that p-tau positively to t-tau in both female (γ=0.656, p<0.0001) and male (γ=0.504, p<0.0001) dementia patients.

The correlation of glycans with MMSE scores and CSF biomarker levels were compared (Table 2). The level of peak 9 had no correlation with MMSE score nor CSF biomarker levels both in female and male patients, while only negatively correlated to the level of peak 6 in the female patients (γ=-0.611, p<0.0001). The level of peak 6 (NA2F) showed no correlation with MMSE score, but most correlation with the CSF markers in female patients. The level of peak 6 was positively correlated to the CSF level of Aβ-42 (γ=0.387, p<0.022) and negatively to the level of p-tau protein (γ=-0.407, p<0.015) in the female dementia patients. No significant correlation was found between peak 6 and t-tau protein.

**Table 2. The correlation coefficient between glycan markers and CSF markers and MMS**

| | | MMSE | t-tau | Aβ-42 | p-tau | peak6 | peak9 |
|---|---|---|---|---|---|---|---|
| MMSE Pearson Correlation | | 1 | 0.045 | 0.044 | -0.059 | 0.286 | -0.182 |
| | Sig. (2-tailed) | | 0.813 | 0.819 | 0.758 | 0.091 | 0.288 |
| | | | | | | | |
| t-tau Pearson Correlation | | 0.045 | 1 | -0.081 | .656(**) | -0.31 | 0.011 |
| | Sig. (2-tailed) | 0.813 | | 0.637 | 0.0001 | 0.07 | 0.951 |
| | | | | | | | |
| Aβ-42 Pearson Correlation | | 0.044 | -0.081 | 1 | 0.027 | .387(*) | -0.096 |
| | Sig. (2-tailed) | 0.819 | 0.637 | | 0.874 | 0.022 | 0.582 |
| | | | | | | | |
| p-tau Pearson Correlation | | -0.059 | .656(**) | 0.027 | 1 | -.407(*) | 0.137 |
| | Sig. (2-tailed) | 0.758 | 0.0001 | 0.874 | | 0.015 | 0.433 |
| | | | | | | | |
| peak6 Pearson Correlation | | 0.286 | -0.31 | .387(*) | -.407(*) | 1 | -.611(**) |
| | Sig. (2-tailed) | 0.091 | 0.07 | 0.022 | 0.015 | | 0.0001 |
| | | | | | | | |
| peak9 Pearson Correlation | | -0.182 | 0.011 | -0.096 | 0.137 | -.611(**) | 1 |
| | Sig. (2-tailed) | 0.288 | 0.951 | 0.582 | 0.433 | 0.0001 | |
| | | | | | | | |

| | | | | | | | |
|---|---|---|---|---|---|---|---|
| ** Correlation is significant at the 0.01 level (2-tailed). * Correlation is significant at the 0.05 level (2-tailed). | | | | | | | |

### Example 5: Comparison of glycan profiles of wild type mice with R406W mutant Tau transgenic mice

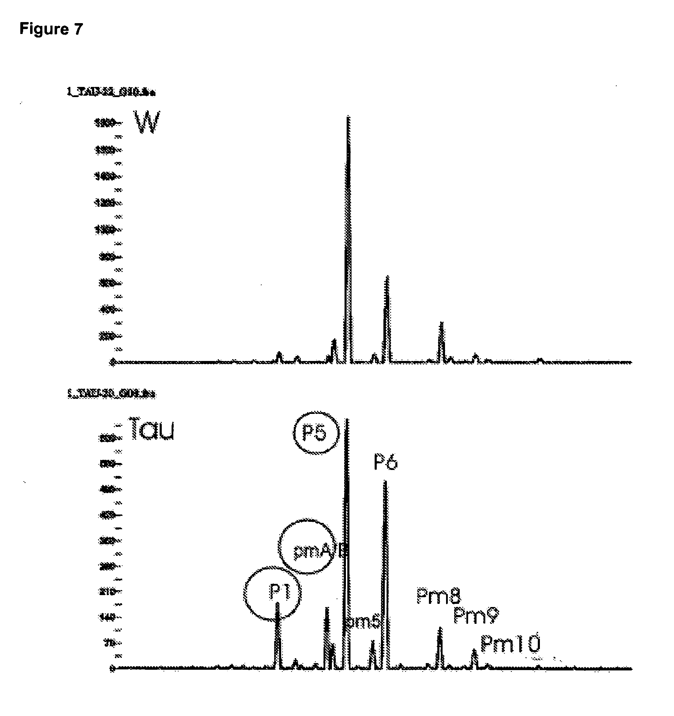

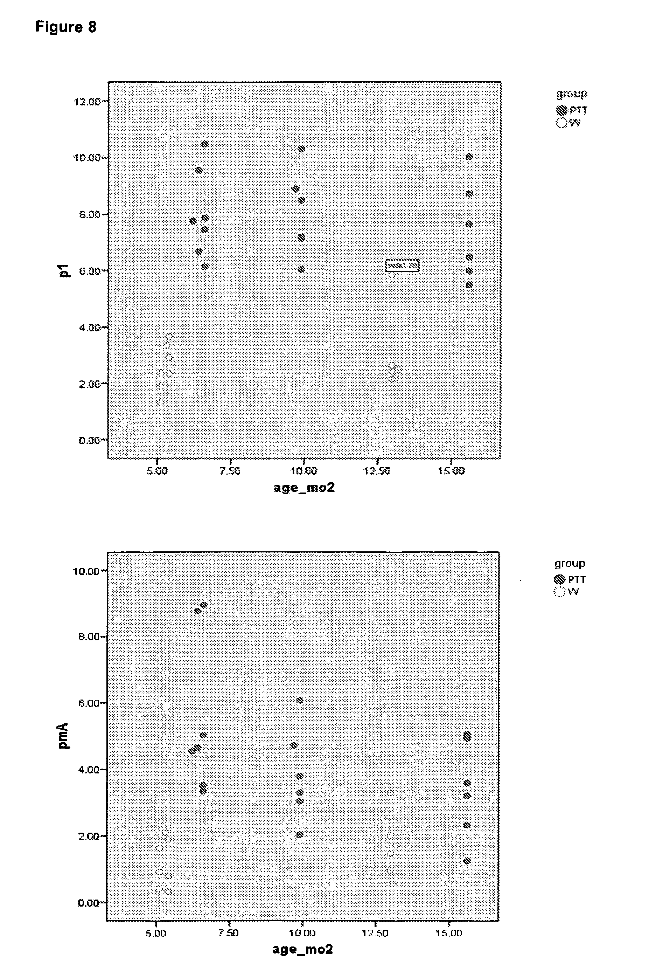

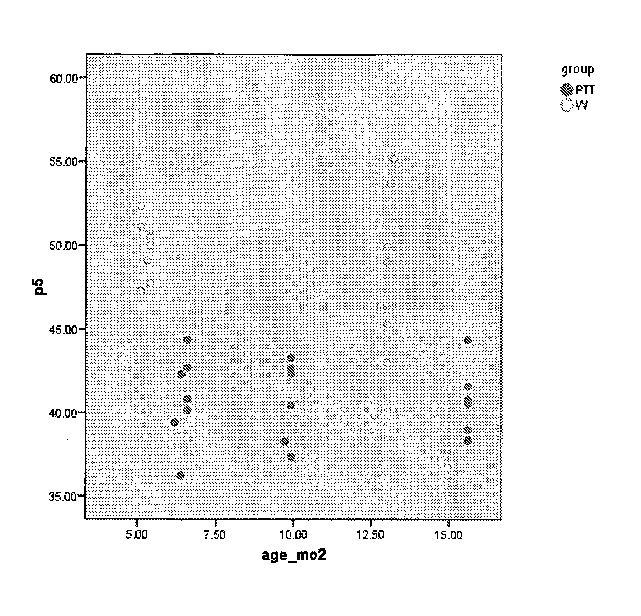

Samples from 13 wild type mice and 21 R406W mutant transgenic mice ((Zhang et al., 2004) (2 different age groups for wild type, 3 different age groups for the transgenic mice) were analyzed. A typical profile for wild type and Tau transgenic mice, with indication of the peaks, is given in Figure 7. Both wild type and tau mice peaks show some shift with age and/or evolution of the disease, but at all ages, there is a significant increase in P1 and pmA, and a significant decrease of P5 in tau transgenic mice, compared with the wild type (Figure 8). One of those peaks, or a combination thereof, can be used to judge the severity of the Alzheimer disease, and/or to follow the effect of a treatment and/or to screen the efficiency of an anti Alzheimer compound.

### REFERENCES

- Akasaka-Manya, K., Manya, H., Sakurai, Y., Wojczyk, B.S., Spitalnik, S.L. and Endo, T. Increased bisecting and core-Fucosylated N-glycans on mutant human amyloid precursor proteins. Glycoconj. J. 25, 775-786 (2008)
- American Psychiatric Association, 1994. DSM-IV: Diagnostic and Statistical Manual of Mental Disorders, fourth ed. American Psychiatric Association, Washington, DC.
- Barkhof, F., et al. The significance of medial temporal lobe atrophy: a postmortem MRI study in the very old. Neurology 69, 1521-1527 (2007).
- Blasko, I., et al. Measurement of thirteen biological markers in CSF of patients with Alzheimer's disease and other dementias. Dementia and geriatric cognitive disorders 21, 9-15 (2006).
- Blasko, I., et al. Conversion from cognitive health to mild cognitive impairment and Alzheimer's disease: prediction by plasma amyloid beta 42, medial temporal lobe atrophy and homocysteine. Neurobiology of aging 29, 1-11 (2008).
- Blennow, K., de Leon, M.J. & Zetterberg, H. Alzheimer's disease. Lancet 368, 387-403 (2006).
- Braak, H. & Braak, E. Neuropathological stageing of Alzheimer-related changes. Acta neuropathologica 82, 239-259 (1991).
- Brand, J.P., et al. An adaptive alpha spending algorithm improves the power of statistical inference in microarray data analysis. Bioinformation 1, 384-389 (2007).
- Buerger, K., et al. Differential diagnosis of Alzheimer disease with cerebrospinal fluid levels of tau protein phosphorylated at threonine 231. Archives of neurology 59, 1267-1272 (2002).
- DeCarli, C., Kaye, J.A., Horwitz, B. & Rapoport, S.I. Critical analysis of the use of computer-assisted transverse axial tomography to study human brain in aging and dementia of the Alzheimer type. Neurology 40, 872-883 (1990).
- De Leon, M.J., et al. Frequency of hippocampal formation atrophy in normal aging and Alzheimer's disease. Neurobiologyofaging 18, 1-11 (1997).
- Dewachter, I, van Dorpe, J., Spittaels, K., Tesseur, I., Van Den Haute, C., Moechars, D., Van Leuven, F. Modelling Alzheimer's disease in trangenic mice: effect of age and of presenilin1 on amyloid biochemlistry and pathology in APP/London mice. Exp Gerontol. 35, 831-841 (2000).
- Dubois, B., et al. Research criteria for the diagnosis of Alzheimer's disease: revising the NINCDS-ADRDA criteria. Lancet neurology 6, 734-746 (2007).
- Engelborghs, S., et al. No association of CSF biomarkers with APOEepsilon4, plaque and tangle burden in definite Alzheimer's disease. Brain 130, 2320-2326 (2007a).
- Engelborghs, S., et al. Diagnostic performance of a CSF-biomarker panel in autopsy-confirmed dementia. Neurobiology of aging (2007b).
- Folstein, M.F., Folstein, S.E. & McHugh, P.R. "Mini-mental state". A practical method for grading the cognitive state of patients for the clinician. Journal of psychiatric research 12, 189-198 (1975).
- Frisoni, G.B., et al. Radial width of the temporal horn: a sensitive measure in Alzheimer disease. Ajnr 23, 35-47 (2002).
- Fujigasaki, H., et al. CAG repeat expansion in the TATA box-binding protein gene causes autosomal dominant cerebellar ataxia. Brain 124, 1939-1947 (2001).
- Galton, C. Alzheimer's disease from basic research to clinical applications. Journal of neurology, neurosurgery, and psychiatry 68, 123B (2000).
- Growdon, J.H. To tap or not to tap: cerebrospinal fluid biomarkers of Alzheimer's disease. Annals of neurology 44, 6-7 (1998).
- Hampel, H., Goemitz, A. & Buerger, K. Advances in the development of biomarkers for Alzheimer's disease: from CSF total tau and Abeta(1-42) proteins to phosphorylated tau protein. Brain research bulletin 61, 243-253 (2003).
- Heckemann, R.A., et al. Automatic volumetry on MR brain images can support diagnostic decision making. BMC medical imaging 8, 9 (2008).
- Hoehn, M.M. & Yahr, M.D. Parkinsonism: onset, progression and mortality. Neurology 17, 427-442 (1967).
- Ibach, B., et al. Cerebrospinal fluid tau and beta-amyloid in Alzheimer patients, disease controls and an age-matched random sample. Neurobiology of aging 27, 1202-1211 (2006).
- Jackson, M. & Lowe, J. The new neuropathology of degenerative frontotemporal dementias. Acta neuropathologica 91, 127-134 (1996).
- Jellinger, K.A. The neuropathological diagnosis of Alzheimer disease. Journal of neural transmission 53, 97-118 (1998).
- Knopman, D.S., et al. Practice parameter: diagnosis of dementia (an evidence-based review). Report of the Quality Standards Subcommittee of the American Academy of Neurology. Neurology 56, 1143-1153 (2001).
- Kosaka, K., Tsuchiya, K. & Yoshimura, M. Lewy body disease with and without dementia: a clinicopathological study of 35 cases. Clinical neuropathology 7, 299-305 (1988).
- Kukuruzinska, M.A. & Lennon, K. Protein N-glycosylation: molecular genetics and functional significance. Crit Rev Oral Biol Med 9, 415-448 (1998).
- Laroy, W, Contreras, R. and Callewaert, N. glycome mapping on DNA sequencing equipment. Nature Protocols 1, 397-405,2006
- Liu, X.E., et al. N-glycomic changes in hepatocellular carcinoma patients with liver cirrhosis induced by hepatitis B virus. Hepatology 46, 1426-1435 (2007).
- Liu, Y., Studzinski, C., Beckett, T., Guan, H., Hersh, M.A., Murphy, M.P., Klein, R and Hersh, L.B. Expression of neprilysin in skeletal muscle reduces amyloid burden in a transgenic mouse model of Alzheimer disease. Mol. Ther. (2009)
- Maddalena, A., et al. Biochemical diagnosis of Alzheimer disease by measuring the cerebrospinal fluid ratio of phosphorylated tau protein to beta-amyloid peptide42. Archives of neurology 60, 1202-1206 (2003).
- Markesbery,W.R., 1998. Neuropathology of Dementing Disorders. Arnold, London.
- Mattman, A., et al. Regional HmPAO SPECT and CT measurements in the diagnosis of Alzheimer's disease. The Canadian journal of neurological sciences 24, 22-28 (1997).
- McKhann, G., et al. Clinical diagnosis of Alzheimer's disease: report of the NINCDS-ADRDA Work Group under the auspices of Department of Health and Human Services Task Force on Alzheimer's Disease. Neurology 34, 939-944 (1984).
- McKeith, I.G., et al. Consensus guidelines for the clinical and pathologic diagnosis of dementia with Lewy bodies (DLB): report of the consortium on DLB international workshop. Neurology 47, 1113-1124 (1996).
- Nagy, Z., et al. Relationship between clinical and radiological diagnostic criteria for Alzheimer's disease and the extent of neuropathology as reflected by 'stages': a prospective study. Dementia and geriatric cognitive disorders 10, 109-114 (1999).
- Neary, D., et al. Frontotemporal lobar degeneration: a consensus on clinical diagnostic criteria. Neurology 51, 1546-1554 (1998).
- Pickut, B.A., et al. Discriminative use of SPECT in frontal lobe-type dementia versus (senile) dementia of the Alzheimer's type. J Nucl Med 38, 929-934 (1997).
- Pype, S., Moechars, D., Dillen, L., and Mercken, M. Characterization of amyloid beta peptides from brain extracts of transgenic mice overexpressing the London mutat of human amyloid precursor protein. J Neurochem. 84, 602-609 (2003).
- Raman, R., Raguram, S., Venkataraman, G., Paulson, J.C. & Sasisekharan, R. Glycomics: an integrated systems approach to structure-function relationships of glycans. Nat Methods 2, 817-824 (2005).
- Roman, G.C., et al. Vascular dementia: diagnostic criteria for research studies. Report of the NINDS-AIREN International Workshop. Neurology 43, 250-260 (1993).
- Selkoe, D.J. & Schenk, D. Alzheimer's disease: molecular understanding predicts amyloid-based therapeutics. Annual review of pharmacology and toxicology 43, 545-584 (2003).
- Tang, J., Song, M., Wang, Y., Fan, X., Xu, H. and Bai, Y. Noggin and BMP4 co-modulate adult hippocampal neurogenesis in the APP(swe)/PS1(DeltaE9) transgenic mouse model of Alzheimer disease. Biochem. Biophys. Res. Commun. (2009)
- Vanhooren, V., et al. N-glycomic changes in serum proteins during human aging. Rejuvenation Res 10, 521-531 a (2007).
- Vanhooren, V., Laroy, W., Libert, C. & Chen, C. N-Glycan profiling in the study of human aging. Biogerontology (2008).
- van Marum, R.J. Current and future therapy in Alzheimer's disease. Fundamental & clinical pharmacology 22, 265-274 (2008).
- Waldemar, G., et al. Recommendations for the diagnosis and management of Alzheimer's disease and other disorders associated with dementia: EFNS guideline. Eur J Neurol 14, e1-26 (2007).
- Weber, T. Clinical and laboratory diagnosis of Creutzfeldt-Jakob disease. Clinical neuropathology 19, 249-250 (2000).
- Wilson, R.J. Towards a cure for dementia: the role of axonal transport in Alzheimer's disease. Science progress 91, 65-80 (2008).
- Zhang, B., Higuchi, M., Yoshimaya, Y., Ishihara, T., Forman, M.S., Martinez, D., Joyce, S., Trojanowski, J.Q. and Lee, V.M.Y. Retarded axonal transport of R406W mutant Tau in transgenic mice with a neurodegenerative tauopathy. J. Neuroscience 24, 4657-4667(2004).

## Claims

1. A method for the detection of Alzheimer's disease in a female human subject, comprising the determination of the concentration of bigalactosylated, core-α-1,6-fucosylated biantennary glycan structures in serum and comparing said concentration with the concentration of a healthy reference subject.

2. The method according to claim 1, whereby said method is used to test the efficiency of a candidate compound against Alzheimer's.

## Patentansprüche

1. Verfahren zur Detektion der Alzheimer-Krankheit bei einem weiblichen menschlichen Subjekt, umfassend die Bestimmung der Konzentration von bigalactosylated, Core-alpha-1,6-fucosylierten biantennären Glykanstrukturen im Serum und Vergleichen der genannten Konzentration mit der Konzentration eines gesunden Referenzsubjekt.

2. Verfahren nach Anspruch 1, wobei das genannte Verfahren verwendet wird zum Testen der Effizienz einer Kandidatenverbindung gegen Alzheimer-Krankheit.

## Revendications

1. Procédé pour la détection de la maladie d'Alzheimer chez un sujet humain féminin, comprenant la détermination de la concentration de structures glycanniques biantennaires bigalactosylés alpha-1,6-fucosylés au niveau du noyau dans le sérum et en comparant ladite concentration avec la concentration d'un objet de référence en bonne santé.

2. Procédé selon la revendication 1, dans lequel ledit procédé est utilisé pour tester l'efficacité d'un composé candidat contre la maladie d'Alzheimer.
